# EUROPÄISCHE PATENTANMELDUNG

(11) **EP 4 488 376 A1**
(43) Veröffentlichungstag der Anmeldung: **08.01.2025**
(21) Anmeldenummer: 23184207.1
(22) Anmeldetag: 07.07.2023
(51) Int. Cl.: C12P 19/02, C12N 9/04

(54) **VERFAHREN ZUR HERSTELLUNG EINER WÄSSERIGEN LÖSUNG ENTHALTEND L-SORBOSE**

(71) Anmelder: Annikki GmbH, 8074 Raaba-Grambach (AT)
(72) Erfinder: Staunig, Nicole, 8074 Raaba-Grambach (AT); Dupont, Maria, 8074 Raaba-Grambach (AT)
(74) Vertreter: Schwarz & Partner Patentanwälte GmbH

(57) **Zusammenfassung**

Verfahren zur Herstellung einer wässerigen Lösung enthaltend L-Sorbose, indem D-Glucose, welche in einer wässerigen Lösung gelöst vorliegt, durch Behandeln mit einer NAD(P)H-abhängigen Oxidoreduktase (Xylose-Reduktase) *in vitro* zu D-Sorbitol reduziert wird, wonach das D-Sorbitol mit einer NAD(P)⁺-abhängigen Oxidoreduktase (Mannitol-Dehydrogenase) *in vitro* zu L-Sorbose oxidiert wird, wonach die beiden Oxidoreduktasen abgetrennt werden.

## Beschreibung

Die vorliegende Erfindung betrifft die Herstellung einer wässerigen Lösung von L-Sorbose.

### Hintergrund der Erfindung

Das Monosaccharid L-Sorbose gehört zur Gruppe der Ketohexosen und ist ein C5-Epimer der D-Fructose. L-Sorbose kann als kalorienarmes Süßungsmittel eingesetzt werden (Shintani, 2019) und dient des Weiteren als Ausgangsstoff für die enzymatische Synthese von seltenen Zuckern und Zuckeralkoholen wie L-Tagatose (Itoh et al., 1996), L-Galactose (Leang et al., 2004) oder L-Iditol (Vongsuvanlert & Tani, 1988). In einer aktuellen Studie konnten die antitumoralen Eigenschaften von L-Sorbose, die die Apoptose von Krebszellen einleitet, nachgewiesen werden (Xu et al., 2023).

Darüber hinaus ist L-Sorbose ein Intermediat in der Reichstein-Synthese von Vitamin C (L-Ascorbinsäure). Die Route startet von D-Glucose, welche zuerst zum Zuckeralkohol D-Sorbitol reduziert wird (siehe unten). D-Sorbitol wird danach mikrobiell zur L-Sorbose oxidiert. Durch Behandeln mit KMnO₄ als Oxidationsmittel erhält man 2-Keto-L-gulonsäure (unter Verwendung von Acetal-Schutzgruppen), die durch Zugabe von Säure zur L-Ascorbinsäure umgewandelt wird (Reichstein & Grüssner, 1934). L-Sorbose kann darüber hinaus auch mikrobiell oder mit molekularem Sauerstoff an Pt- oder Pd-basierten Katalysatoren zur 2-Keto-L-gulonsäure oxidiert werden (Sugisawa et al., 1990; Bronnimann et al., 1994).

Zur Herstellung von L-Sorbose sind einige chemische Verfahren bekannt. So kann beispielsweise D-Glucose zu L-Sorbose unter Verwendung von Pure Silicate Zeolite Beta mit Lewis-sauren Ti⁴⁺-Zentren isomerisiert werden. Die beobachteten Ausbeuten an L-Sorbose liegen allerdings bei max. 12% (w/w) bei Einsatz einer 1% (w/w) D-Glucose-Lösung sowie einer Reaktionstemperatur von 100 °C. Darüber hinaus werden D-Fructose und D-Mannose als Nebenprodukte gefunden (Gounder & Davis, 2013). Diese Methode wurde auch in US 9255120 B2 und US 11292806 B2 patentiert.

L-Sorbose kann auch durch elektrochemische Oxidation von D-Sorbitol an Pt-Elektroden mit p-Block-Metallen (Bi, Sb, Pb, Sn, In) als Promotoren erhalten werden, jedoch wird D-Fructose als Nebenprodukt gebildet (Kwon et al., 2014).

Zur mikrobiellen Oxidation von D-Sorbitol werden hauptsächlich Essigsäurebakterien wie *Gluconobacter* oder *Acetobacter* verwendet (Shintani, 2019; Zebiri et al., 2011; WO 2000/049133 A1; EP 0233050 B1; EP 0273648 B1; EP 0955358 B1; EP 1153120 A1; EP 0199548 A2; US 6664082 B1; US 4945048; JP H07102127 B2; KR 820001130 B1).

US 4904588 beschreibt verschiedene *Gluconobacter (sub)oxydans-Stämme,* die bis zu 300 g/l D-Sorbitol innerhalb von 24 h bzw. 200 g/l D-Sorbitol innerhalb von 40 h vollständig zu L-Sorbose oxidieren können. Liu et al. (2022) verwendeten einen engineerten G. oxydans-Stamm zum Umsatz von 300 g/l D-Sorbitol zu 298,61 g/l L-Sorbose in 100 h.

Die für die Oxidation von D-Sorbitol zu L-Sorbose verantwortlichen Enzyme sind die membrangebundene o-Sorbitol-Dehydrogenase (mSLDH; EC 1.1.99.21), die als Kofaktor entweder Pyrrolochinolinchinon (PQQ) oder Flavin-Adenin-Dinukleotid (FAD) (Toyama et al., 2005; Soemphol et al., 2007; Yang et al., 2008) aufweist, die NADP-abhängige o-Sorbitol-Dehydrogenase aus G. *oxydans* (GoSLDH) (Kim et al., 2019), die NADP-abhängige L-Sorbose-Reduktase (SR; EC 1.1.1.289) (Sugisawa et al., 1991; Shinjoh et al., 2002; Kubota et al., 2011) sowie die Mannitol-2-Dehydrogenase (MDH; EC 1.1.1.67) aus *Pseudomonas fluorescens* (Kavanagh et al., 2002) oder *Aspergillus fumigatus* (Krahulec et al., 2011).

KR 102060253 B1 beschreibt einen rekombinanten E. coli-Stamm mit exprimierter GoSLDH und einer NAD(P)H-Oxidase (zur Kofaktor-Regeneration) aus *Lactobacillus reuteri,* welche 10 mM (1,82 g/l) D-Sorbitol zu 92% umsetzte.

Die enzymatische Oxidation von D-Sorbitol kann auch in zellfreien Verfahren *(in vitro)* durchgeführt werden. WO 2014/171635 A1 beschreibt eine GoSLDH, mit der 10 mM (1,82 g/l) D-Sorbitol innerhalb von 3 h zu 7,5 mM L-Sorbose umgesetzt wurden. Kim et al. (2016) verwendeten ebenfalls eine GoSLDH aus G. *oxydans* G624 zur Oxidation von 50 mM (9,11 g/l) D-Sorbitol zu L-Sorbose (47% Umsatz in 3 h). In beiden beschriebenen Verfahren wurde der Kofaktor NADP in stöchiometrischen Mengen verwendet, d.h. es wurde kein Regenerationssystem für den Kofaktor verwendet.

EP 0955358 A2 beschreibt genetisch modifizierte *Gluconobacter-* oder *Acetobacter*-Mutanten mit fehlender L-Sorbose-Reduktase-Aktivität. Mit der Mutante SR3 wurden 80 g/l D-Sorbitol innerhalb von 24 h zu 75 g/l L-Sorbose oxidiert, wobei die L-Sorbose-Konzentration im Medium nach 5 Tagen auf 70 g/l absank. Im Vergleichsstamm *(G. suboxydans* IFO 3291) mit intakter L-Sorbose-Reduktase-Aktivität wurden nach 24 h nur ca. 40 g/l L-Sorbose gefunden, die dann größtenteils weiter verstoffwechselt wurde.

Bei Verwendung einer mSLDH (aus G. *suboxydans* IFO 3255) muss zur Oxidation von D-Sorbitol (38 mM = 6,95 g/l) ein Elektronenakzeptor wie Phenazin-Methosulfat (7,7 mM) zugesetzt werden (Bildungsrate von L-Sorbose: 1,3 mg/h), wie in EP 0728840 B1 sowie US 5747301 beschrieben.

Der Zuckeralkohol D-Sorbitol kommt auch in geringen Mengen natürlich z.B. in den Früchten der Vogelbeere *(Sorbus aucuparia* L.) vor (Lohmar, 1957), wird aber zum größten Teil chemisch durch

Hydrierung an Nickel-Oberflächen hergestellt. Industriell häufig eingesetzte Raney-NickelKatalysatoren zeichnen sich durch ihre hohe Aktivität und ihren niedrigen Preis aus, leiden jedoch an Auswaschung von Nickel im Laufe des Prozesses (Jonas & Silveira, 2004; Zhao et al., 2020; Garcia et al., 2021), wodurch Nickel-Rückstände in nachfolgenden Reinigungsschritten aus dem Produkt entfernt werden müssen. Der Kontakt mit Nickel kann eine Reihe von Erkrankungen wie Allergien, Herzkreislauf- und Nierenerkrankungen, Lungenfibrose sowie Lungen- und Nasenkrebs auslösen (Genchi et al., 2020).

Biotechnologische Verfahren zur Herstellung von D-Sorbitol sind ebenfalls bekannt.

So kann D-Sorbitol fermentativ mit *Zymomonas mobilis* hergestellt werden - das verantwortliche Enzym ist die Glucose-Fructose-Oxidoreduktase (GFOR, EC 1.1.99.28), welche Mischungen aus D-Glucose und D-Fructose zu D-Gluconolacton (oder D-Gluconat durch enzymatische Hydrolyse) und D-Sorbitol umsetzt (Barrow et al., 1984; Zachariou & Scopes, 1986). Verfahren mit Z. *mobilis* sind beispielsweise in US 4755467, US 5190869, EP 0427150 A1 sowie EP 0212517 A2 beschrieben.

EP 0132557 B1 legt ein Verfahren zur Umsetzung einer D-Glucose/D-Fructose-Mischung mittels immobilisierter Glucose-Dehydrogenase aus *Bacillus megaterium* und Sorbitol-Dehydrogenase aus Schafsleber zu D-Gluconat und Sorbitol offen. Ikemi et al. beschrieben einen ein ähnliches System, wobei nur D-Glucose als Substrat eingesetzt wurde. Die Reduktion erfolgte mit einer Aldose-Reduktase aus *Candida tropicalis* IAM 12202, wobei die Regeneration des Kofaktors NADPH mit einer Glucose-Dehydrogenase bewerkstelligt wurde (lkemi et al., 1990a; Ikemi et al., 1990b).

Durch die Aldol-Reaktion von L-Glyceraldehyd und Dihydroxyacetonphosphat (DHAP), katalysiert durch eine Fructose-1,6-diphosphat-Aldolase oder eine Tagatose-1,6-diphosphat-Aldolase, mit anschließender Abspaltung der endständigen Phosphat-Gruppe wird eine Mischung aus L-Psicose und L-Sorbose erhalten. Die Reaktionskaskade kann sowohl *in vitro* als auch fermentativ in einem engineerten *Corynebacterium glutamicum-Stamm* durchgeführt werden (Yang et al., 2015). Yang et al. (2016) entwickelten die Reaktionskaskade weiter, sodass auch Glycerin als Startmaterial für die fermentative Herstellung von L-Sorbose verwendet werden kann. Das Herzstück der Kaskade ist ebenfalls die Aldol-Reaktion von L-Glyceraldehyd und DHAP.

L-Sorbose kann aber auch direkt aus Mischungen von D-Glucose und D-Fructose hergestellt werden. Im ersten Schritt wird D-Fructose mit permeabilisierten Z. *mobilis-Zellen* zu D-Sorbitol reduziert, welches von *G. suboxydans* in einem zweiten Schritt zu L-Sorbose oxidiert wird. Der Oxidationsschritt durch die von Z. *mobilis* aus D-Glucose gebildete D-Gluconsäure inhibiert, weswegen der Prozess als zweistufiges Fed-Batch-Verfahren durchgeführt werden muss (Kim et al., 1994).

Die meisten biotechnologischen Verfahren für die Produktion von D-Sorbitol starten von D-Fructose, einzig das Verfahren von Ikemi et al. (lkemi et al., 1990a; Ikemi et al., 1990b) startet vom günstigeren Rohstoff D-Glucose, der auch zur großtechnischen Herstellung von D-Sorbitol durch Hydrierung verwendet wird. Der Nachteil an dem Verfahren ist allerdings, dass die Hälfte der D-Glucose zu D-Gluconsäure oxidiert werden muss, um die nötigen Reduktionsäquivalente zur Herstellung von D-Sorbitol bereitzustellen.

Für L-Sorbose ist nach derzeitigem Stand der Technik kein Eintopf-Verfahren, also ein Verfahren ohne Isolierung von Zwischenprodukten, ausgehend von D-Glucose beschreiben.

Für D-Fructose (das C5-Epimer von L-Sorbose) wiederum sind in der Literatur enzymatische Verfahren (z.B. US 11339415 B2) bekannt, die über das Intermediat D-Sorbitol ablaufen. D-Glucose wurde zuerst mit einer Xylose-Reduktase aus *Candida tropicalis* in Kombination mit einer Alkoholdehydrogenase (ADH) aus *Lactobacillus kefir* zur Kofaktor-Regeneration (oxidiert 2-Propanol zu Aceton) zu D-Sorbitol reduziert, wobei beide Enzyme in Form von Zell-Lysaten zugesetzt wurden. Nach Deaktivierung der Enzyme bei 60 °C unter Anlegen eines Vakuums (zur Entfernung von 2-Propanol und Aceton) wurde der Oxidationsschritt zu D-Fructose mit einer Sorbitol-Dehydrogenase aus *Bacillus subtilis* durchgeführt, wobei die Kofaktor-Regeneration entweder mit einer Lactat-Dehydrogenase aus Kaninchenmuskel (und Pyruvat als Kosubstrat) oder mit einer NADH-Oxidase aus *Leuconostoc mesenteroides* bewerkstelligt wird.

Hier setzt nun die Aufgabe der vorliegenden Erfindung an. Die Erfindung möchte ein effizientes Verfahren zur Herstellung einer wässerigen Lösung von L-Sorbose mit hoher Produktkonzentration und hohem Umsatz ausgehend von D-Glucose zur Verfügung stellen, welches im Eintopf-Verfahren durchgeführt werden kann und bei dem keine Deaktivierung zwischen dem Reduktions- und Oxidationsschritt durchgeführt werden muss.

### Detaillierte Beschreibung der Erfindung

Die vorliegende Erfindung besteht darin, dass D-Glucose, welche in einer wässerigen Lösung gelöst vorliegt, durch Behandeln mit einer NAD(P)H-abhängigen Oxidoreduktase *in vitro* zu D-Sorbitol reduziert wird, wonach das D-Sorbitol mit einer NAD(P)⁺-abhängigen Oxidoreduktase *in vitro* zu L-Sorbose oxidiert wird, wonach die beiden Oxidoreduktasen abgetrennt werden.

Eine bevorzugte Variante des erfindungsgemäßen Verfahrens besteht darin, dass der durch die Reduktion entstandene oxidierte Kofaktor NAD(P)⁺ mittels einer Alkoholdehydrogenase und einem sekundären Alkohol unter Bildung eines Ketons reduziert wird. Verfahren zur Regenerierung von Kofaktoren sind z.B. aus EP 2812439 B1 bekannt.

Als sekundärer Alkohol wird insbesondere 2-Propanol eingesetzt.

Der durch die Oxidation entstandene reduzierte Kofaktor NAD(P)H wird am besten mittels einer Oxidase und Sauerstoff zu NAD(P)⁺ oxidiert.

Das erfindungsgemäße Verfahren kann als Eintopf-Reaktion ohne Isolierung von Zwischenprodukten durchgeführt werden.

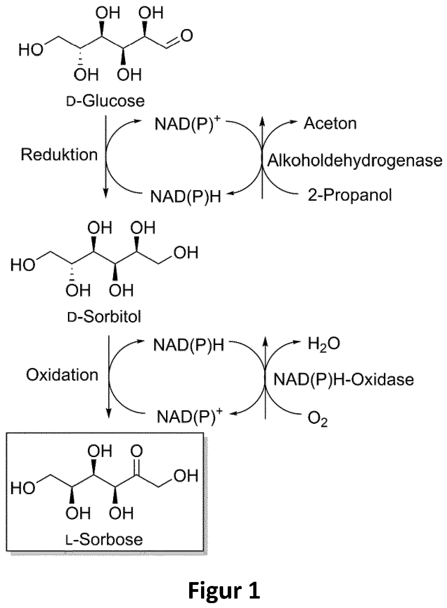

In der beiliegenden Figur 1 ist das Reaktionsschema des erfindungsgemäßen Verfahrens wiedergegeben.

Die besonders bevorzugte Konzentration von D-Glucose beträgt 50 - 250 g/l.

Der besonders bevorzugte Temperaturbereich für Reduktionsschritt liegt zwischen 25 und 55 °C, für den Oxidationsschritt zwischen 20 und 30 °C.

Der besonders bevorzugte pH-Bereich beider Schritte liegt zwischen 7,0 und 9,0.

Bei einer weiteren, bevorzugten Variante des erfindungsgemäßen Verfahrens liegen die Enzyme in einer Suspension, im Homogenat und/oder im Lysat der entsprechenden, sie bildenden Zellen vor, wobei Lysate besonders bevorzugt sind.

Suspension bedeutet in diesem Kontext eine Suspension von *resting cells.* Diese werden nach der Kultivierung geerntet (vom Nährmedium abgetrennt) und in einem geeigneten Puffersystem suspendiert. Im Gegensatz zu fermentativen Verfahren, bei denen auch mit ganzen Zellen gearbeitet wird, können die *resting cells* aufgrund des Fehlens von Kohlenstoff-Quellen und Nährstoffen nicht mehr wachsen, sondern dienen nur der Umsetzung von Substraten (Lin & Tao, 2017). Homogenat steht in diesem Kontext für eine physikalisch und/oder chemisch behandelte Suspension (z.B. mittels Druckes, Lysozym oder Ultraschall behandelt), wobei die Zellbestandteile aus den Zellen freigesetzt werden. Ein Lysat wird erhalten, wenn die unlöslichen Zellbestandteile des Homogenats beispielsweise durch Filtration oder Zentrifugation entfernt werden (siehe *Produktion der Enzyme* & *Herstellung der Lysate* für Details).

In einer weiteren Variante können die Enzyme auch auf einem festen Trägermaterial immobilisiert sein.

Die Abtrennung der Enzyme kann z.B. durch Zentrifugieren oder Ultrafiltration bewerkstelligt werden.

Das zur Reduktion von D-Glucose verwendete Enzym stammt aus der Gruppe der Oxidoreduktasen, wobei eine Xylose-Reduktase besonders bevorzugt ist.

Das zur Oxidation von D-Sorbitol verwendete Enzym stammt aus der Gruppe der Oxidoreduktasen, wobei eine Mannitol-Dehydrogenase besonders bevorzugt ist.

Die zur Kofaktor-Regeneration eingesetzte Alkoholdehydrogenase (ADH) kann aus einer der Gruppen EC 1.1.1.1 (NAD-abhängige ADH) und EC 1.1.1.2 (NADP-abhängige ADH) stammen.

Die zur Kofaktor-Regeneration eingesetzte NAD(P)H-Oxidase kann aus einer der Gruppen EC 1.6.3.1 (NAD(P)H-Oxidase (H₂O₂-bildend)), EC 1.6.3.2 (NAD(P)H-Oxidase (H₂O-bildend)), EC 1.6.3.3 (NADH-Oxidase (H₂O₂-bildend)) sowie EC 1.6.3.4 (NADH-Oxidase (H₂O-bildend)) stammen, wobei die H₂O-bildenden Klassen besonders bevorzugt sind.

Die erfindungsgemäß eingesetzte Oxidoreduktase zur Bildung von D-Sorbitol aus D-Glucose ist vorzugsweise eine Xylose-Reduktase. Es hat sich überraschenderweise gezeigt, dass Xylose-Reduktasen in der Lage sind, D-Glucose in Anwesenheit des Kofaktors NAD(P)H zu D-Sorbitol umzusetzen. Dementsprechend betrifft ein weiterer Aspekt der vorliegenden Erfindung die Verwendung von Xylose-Reduktasen zur Reduktion von D-Glucose zu D-Sorbitol in Anwesenheit des Kofaktors NAD(P)H.

Die Oxidoreduktase zur Bildung von D-Sorbitol aus D-Glucose umfasst vorzugsweise eine Aminosäuresequenz, welche ausgewählt ist aus der Gruppe bestehend aus:
i) einer Aminosäuresequenz, die eine Identität zu SEQ ID Nr. 1 oder 3 von mindestens 80% aufweist,
ii) einer Aminosäuresequenz, die durch eine Nukleinsäure kodiert wird, die eine Identität zu SEQ ID Nr. 2 oder 4 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 2 oder 4 bindet, wobei stringente Bedingungen vorzugsweise einen oder mehrere Waschschritte bei 65°C und einer Salzkonzentration von 0,1x bis 2x SSC umfassen.

SEQ ID Nr. 1: 
MATPTIKLNSGYDMPLVGFGLWKVNRETCADQVYEAIKAGYRLFDGACDYGNEVEAGQGVARAIKEGIVKR
EDLFIVSKLWNTFHEADKVEPIARKQLADWGLDYFDLYLIHFPIALKYVDPAEIYPPGWTGTKKEVEFSNATIQE
TWQAMETLVDKKLTRSIGISNFSAQLIMDLLRYARIRPATLQIEHHPYLTQQALVEYVQKEGIAVTAYSSFGPLS
FLELGHQVAKDTPLLFEHSTVKSIAEKHGKTPAQVLLRWATQRNIAVIPKSNNPGRLAQNLDVTAWDLEPADI
EALSALNKNLRFNNPPSYGLYIPIFA
SEQ ID Nr. 2: 
ATGGCCACGCCTACTATCAAGCTGAACAGCGGCTATGACATGCCCCTGGTGGGCTTTGGTCTGTGGAAG
GTCAACAGGGAAACCTGCGCGGACCAGGTCTACGAGGCTATCAAGGCGGGCTACCGCTTGTTTGACGGT
GCGTGCGACTATGGCAACGAAGTTGAGGCCGGCCAGGGTGTCGCTCGCGCCATCAAGGAAGGCATTGT
GAAGCGTGAGGACCTCTTCATTGTGTCCAAGCTGTGGAACACGTTCCACGAGGCCGACAAGGTCGAGCC
GATCGCGCGGAAGCAGCTGGCCGACTGGGGCCTCGACTACTTTGACCTGTACCTCATCCACTTCCCGATC
GCGCTGAAGTACGTCGACCCGGCCGAGATCTACCCGCCGGGCTGGACGGGCACCAAGAAGGAGGTCGA
GTTCAGCAACGCGACGATCCAGGAGACGTGGCAGGCCATGGAGACCCTGGTCGACAAGAAGCTGACGC
GCAGCATCGGCATCAGCAACTTCAGCGCCCAGCTGATCATGGACCTGCTGCGGTACGCGCGCATCCGCCC
CGCGACCTTGCAGATCGAGCACCACCCGTACCTGACGCAGCAGGCGCTGGTCGAGTACGTGCAGAAGGA
GGGCATCGCCGTGACGGCGTACTCGTCCTTCGGCCCACTGAGCTTCCTGGAACTGGGCCACCAGGTCGCC
AAGGACACGCCGCTGCTCTTCGAGCACTCGACCGTCAAGTCGATCGCCGAGAAGCACGGCAAGACGCCC
GCCCAGGTGCTACTGCGCTGGGCCACCCAGCGCAACATCGCCGTCATCCCCAAGAGCAACAACCCGGGC
CGCCTGGCGCAGAACCTGGACGTGACGGCGTGGGATCTGGAGCCCGCCGACATTGAGGCCTTGAGCGC
GCTGAACAAGAACCTTCGATTCAACAACCCACCTAGCTACGGACTGTACATCCCGATCTTCGCTTAA
SEQ ID Nr. 3: 
MAPQIPNIKLSSGYDMPQVGFGLWKVDRSICADVVYNAIKIGYRLFDGACDYGNEVEAGQGIARAIKEGIVKR
EELFIVSKLWNTFHDGDKVEPIVRKQLADWGIDYFDLYLVHFPVALEYVDPSVRYPPGWFYDGEKEIRPSKATI
QETWTAMESLVEKGLARSIGVSNFQAQLLYDLLRYAKIRPATLQIEHHPFLVQQELLNLAKAEGIAVTAYSSFG
PQSFLEFNMKHAVQLTPLFEDETIKKIAAKYNRPASQVLLRWATQRGLAIIPKSTRPEIMKSNLESIEFDLSEEDI
ATISAFDRGLRFNQPTNYFPTEHLWIFG
SEQ ID Nr. 4:

ATGGCTCCCCAGATCCCCAACATTAAGCTCAGCAGCGGCTATGACATGCCCCAGGTGGGCTTTGGACTGT
GGAAGGTCGACCGCTCGATCTGCGCCGACGTCGTCTACAACGCAATTAAGATTGGCTACCGCCTGTTTGA
CGGCGCTTGCGACTATGGCAACGAAGTCGAGGCCGGCCAGGGCATTGCCCGCGCCATCAAGGAGGGCA
TCGTGAAGCGTGAGGAGCTCTTCATCGTGTCCAAGCTCTGGAACACCTTCCACGACGGCGACAAGGTCG
AGCCCATCGTCCGCAAGCAGCTCGCCGACTGGGGCATTGACTACTTCGACCTCTACCTCGTCCACTTCCCT
GTCGCCCTCGAGTACGTCGACCCCTCGGTCCGCTACCCGCCCGGCTGGTTCTACGATGGCGAGAAGGAG
ATCCGCCCCAGCAAGGCCACCATCCAGGAGACCTGGACCGCCATGGAGTCGCTCGTTGAGAAGGGTCTG
GCCCGCAGCATTGGTGTCTCCAACTTCCAGGCCCAGCTCCTCTACGACCTGCTGCGCTACGCCAAGATCC
GCCCGGCCACCCTTCAGATCGAGCATCACCCCTTCCTCGTCCAGCAGGAGCTGCTCAACCTGGCCAAGGC
CGAGGGCATTGCCGTGACTGCCTACAGCTCATTCGGCCCTCAGAGCTTCCTCGAGTTCAACATGAAGCAC
GCCGTGCAGCTCACCCCGCTCTTCGAGGACGAGACCATCAAGAAGATCGCCGCCAAGTACAACCGTCCTG
CTTCGCAGGTTCTCCTGCGCTGGGCCACTCAGCGCGGTCTGGCCATTATCCCCAAGAGCACGCGCCCCGA

GATCATGAAGTCCAACCTCGAGAGCATCGAGTTCGACCTCAGCGAGGAAGATATTGCCACCATCTCGGCC
TTCGACCGCGGCCTGCGCTTCAACCAGCCCACAAACTACTTCCCCACCGAGCACCTCTGGATCTTTGGCTA
A

Die Oxidoreduktase zur Bildung von D-Sorbitol aus D-Glucose umfasst oder besteht vorzugsweise aus einer Aminosäuresequenz, die eine Identität zu SEQ ID Nr. 1 oder 3 von mindestens 80%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist. Besonders bevorzugt umfasst die erfindungsgemäße Oxidoreduktase zur Bildung von D-Sorbitol aus D-Glucose die Aminosäuresequenz SEQ ID Nr. 1 oder 3 oder besteht aus dieser.

Alternativ umfasst oder besteht die Oxidoreduktase zur Bildung von D-Sorbitol aus D-Glucose vorzugsweise aus einer Aminosäuresequenz, die durch eine Nukleinsäure kodiert wird, die eine Identität zu SEQ ID Nr. 2 oder 4 von mindestens 80%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist. Besonders bevorzugt umfasst die Nukleinsäure, welche die erfindungsgemäße Oxidoreduktase zur Bildung von D-Sorbitol aus D-Glucose kodiert, die Nukleinsäuresequenz SEQ ID Nr. 2 oder 4 oder besteht aus dieser.

Der Begriff "Identität", wie hier verwendet, bezieht sich auf den Prozentsatz der identischen Nukleotid- bzw. Aminosäureübereinstimmungen zwischen mindestens zwei, unter Verwendung eines standardisierten Algorithmus miteinander ausgerichteten Nukleotid- bzw. Aminosäuresequenzen ("Alignment"). Ein solcher Algorithmus kann, in einer standardisierten und reproduzierbaren Weise, Lücken ("Gap") in die verglichenen Sequenzen einfügen, um das Alignment zwischen zwei Sequenzen zu optimieren, und somit einen aussagekräftigeren Vergleich der beiden Sequenzen erreichen.

Die prozentuale Identität zwischen Sequenzen kann unter Verwendung von ein oder mehreren Computeralgorithmen oder im Stand der Technik bekannten oder hierin beschriebenen Programmen bestimmt werden. Erfindungsgemäß wird zur Bestimmung der Identität das durch National Center for Biotechnology Information (NCBI) bereitgestellte Basic Local Alignment Search Tool (BLAST) (Altschul et al., 1990), verwendet. Die BLAST-Software-Reihe enthält verschiedene Programme, einschließlich ein als "BLAST 2 Sequences" genanntes Werkzeug, das für den direkten paarweisen Vergleich von zwei Nukleotid- bzw. Aminosäuresequenzen verwendet wird. "BLAST 2 Sequences" kann auch über die NCBI World Wide Web-Seite im Internet interaktiv abgerufen und verwendet werden. Das blastn-Programm (für Nukleotidsequenzen) verwendet als Vorgaben eine Wortlänge (W) von 28, eine Erwartung (E) von 0,05, M = 1, N = -2 und einen Vergleich beider Stränge. Für Aminosäuresequenzen verwendet das blastp-Programm als Vorgaben eine Wortlänge von 3 und eine Erwartung (E) von 0,05 und die BLOSUM62-Scoring-Matrix (Henikoff & Henikoff, 1989), Alignments (B) von 50, Erwartung (E) von 0,05, M = 1, N =-2.

Alternativ umfasst die Oxidoreduktase zur Bildung von L-Sorbose aus D-Glucose vorzugsweise eine Aminosäuresequenz oder besteht aus dieser, die durch eine Nukleinsäure kodiert wird, die unter stringenten Bedingungen an ein Nukleinsäuremolekül mit der Nukleinsäuresequenz SEQ ID Nr. 2 oder 4 bindet. Wie hierin verwendet, beziehen sich die stringenten Bedingungen auf Bedingungen, unter denen sogenannte spezifische Hybride, jedoch keine unspezifischen Hybride gebildet werden.

Beispielsweise kann die Hybridisierung durch herkömmlich bekannte Verfahren durchgeführt werden, wie die von J. Sambrook et al. in Molecular Cloning, A Laboratory Manual, 2nd Ed., Cold Spring Harbor Laboratory (1989) beschriebenen. Unter stringenter Bedingung ist eine Bedingung gemeint, bei der das Waschen bei 65 °C und einer Salzkonzentration von 0,1x bis 2x SSC durchgeführt wird (wobei unter 1xSSC eine Mischung aus 0,15 M Natriumchlorid/0,015 M Natriumcitrat zu verstehen ist).

Die erfindungsgemäß eingesetzte Oxidoreduktase zur Oxidation von D-Sorbitol zu L-Sorbose ist vorzugsweise eine Mannitol-Dehydrogenase. Es hat sich überraschenderweise gezeigt, dass Mannitol-Dehydrogenasen in der Lage sind, D-Sorbitol in Anwesenheit des Kofaktors NAD(P)⁺ zu L-Sorbose umzusetzen. Dementsprechend betrifft ein weiterer Aspekt der vorliegenden Erfindung die Verwendung von Mannitol-Dehydrogenasen zur Oxidation von D-Sorbitol zu L-Sorbose in Anwesenheit des Kofaktors NAD(P)⁺.

Die Oxidoreduktase zur Bildung von L-Sorbose aus D-Sorbitol umfasst vorzugsweise eine Aminosäuresequenz, welche ausgewählt ist aus der Gruppe bestehend aus:
i) einer Aminosäuresequenz, die eine Identität zu SEQ ID Nr. 5 von mindestens 80% aufweist,
ii) einer Aminosäuresequenz, die durch eine Nukleinsäure kodiert wird, die eine Identität zu SEQ ID Nr. 6 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 6 bindet, wobei stringente Bedingungen vorzugsweise einen oder mehrere Waschschritte bei 65°C und einer Salzkonzentration von 0,1x bis 2x SSC umfassen.

SEQ ID Nr. 5: 
MATKFSLATLDAVKATAGVPNYGRHDLRAGIVHFGVGNFHRAHQAVYLDDLFNLGRDRDWAIIGAGVLPSD
KVMRDKLEAQDFLTTVVEQDNNRTGAHVTGAMIAYLEPGDTPAIVAQLASPLIRIVSLTITEGGYFIDPASGVF
DPAHPAIVEDARDPAAPKTVFGLILAGLAERRAKGIPPFTIMSCDNIPGNGEVTHAAVSGLARLSDPGFADWI
 
DANVAFPNGMVDRITPATGAREIGIVASQYGIDDAWPVFCEEFKQWVLEDRFPQGRPALEEVGVQFVPDVA
PYEHMKIRILNGGHAAIAYPAALLDIHFVHEAMEEPLIRAFLSKLEHDEIIPVIPPVPDTDLKDYYKLIETRFSNPKI
GDTVARLAQDGSNRQPKFILPSTADRLRRGEDVVGLSLVSALWCRYFAGKSDSGKEIVFNDANADRLHAAAV
AAKDDPMAFLALSDIFGDVAQSDLFRRRFAHALKVLWEKGTRATLQLYLDGNLGE
SEQ ID Nr. 6:

ATGGCGACCAAATTCTCCCTTGCCACGCTCGACGCGGTCAAAGCCACGGCCGGCGTCCCGAACTACGGCC
GGCATGACCTTCGGGCCGGCATCGTGCACTTCGGCGTCGGCAATTTCCACCGCGCGCATCAGGCGGTCTA
TCTCGATGACCTTTTCAACCTGGGACGCGATCGCGATTGGGCGATCATCGGCGCGGGTGTCCTGCCATCG
GACAAGGTCATGCGCGACAAGCTCGAAGCGCAGGATTTCCTGACGACGGTGGTCGAGCAGGACAACAA
CCGCACGGGCGCGCATGTCACCGGCGCCATGATCGCCTACCTCGAGCCCGGCGACACGCCCGCGATCGT
GGCGCAGCTCGCGAGCCCGCTCATCCGCATCGTCTCCCTGACGATCACCGAAGGCGGCTATTTCATCGAT
CCGGCATCCGGCGTCTTCGACCCGGCGCATCCGGCGATCGTCGAGGATGCGCGCGATCCGGCGGCTCCA
AAAACCGTCTTCGGCCTCATCCTGGCAGGGCTCGCCGAACGCCGCGCCAAGGGTATTCCGCCGTTCACGA
TCATGTCCTGCGACAATATTCCTGGAAACGGGGAGGTCACCCACGCTGCCGTTTCCGGTCTGGCGCGCCT
TTCCGATCCAGGCTTTGCGGACTGGATCGATGCCAATGTCGCCTTTCCGAACGGCATGGTCGATCGCATC
ACGCCGGCGACCGGTGCCCGCGAGATCGGCATCGTCGCCTCGCAATACGGAATCGACGATGCCTGGCCG
GTCTTTTGCGAGGAATTCAAGCAATGGGTGCTGGAAGACCGCTTCCCGCAAGGCCGCCCGGCGCTGGAA
GAGGTGGGCGTTCAGTTCGTGCCCGACGTGGCGCCGTACGAGCACATGAAGATCCGTATCCTCAACGGC
GGCCATGCGGCGATCGCCTATCCGGCGGCACTGCTCGACATCCATTTCGTTCATGAGGCGATGGAGGAG
CCGTTGATCCGCGCCTTCCTGTCAAAGCTGGAGCACGACGAGATCATCCCGGTGATACCGCCCGTTCCGG
ACACGGATCTGAAGGATTATTACAAGCTCATCGAGACGCGTTTCTCCAATCCGAAGATCGGCGACACGGT
CGCGCGGCTGGCGCAGGACGGCTCCAACCGACAGCCGAAATTCATCCTGCCGTCGACGGCCGACCGCCT
GCGCCGCGGCGAAGACGTCGTCGGGTTGTCGCTCGTTTCGGCGCTGTGGTGCCGCTATTTCGCCGGCAA
GTCGGACAGCGGCAAGGAGATCGTTTTCAACGACGCCAACGCCGACCGGCTGCATGCCGCCGCGGTTGC
AGCGAAGGACGATCCAATGGCGTTCCTTGCGCTTTCCGACATCTTCGGCGATGTCGCGCAATCCGATCTC
TTCCGCCGCCGTTTCGCTCACGCGTTGAAAGTGCTTTGGGAAAAGGGTACGCGTGCCACGCTCCAGCTTT
ATCTGGACGGAAACCTCGGGGAATAA

Die Oxidoreduktase zur Bildung von L-Sorbose aus D-Sorbitol umfasst oder besteht vorzugsweise aus einer Aminosäuresequenz, die eine Identität zu SEQ ID Nr. 5 von mindestens 80%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist. Besonders bevorzugt umfasst die erfindungsgemäße Oxidoreduktase zur Bildung von L-Sorbose aus D-Sorbitol die Aminosäuresequenz SEQ ID Nr. 5 oder besteht aus dieser.

Alternativ, umfasst oder besteht die Oxidoreduktase zur Bildung von L-Sorbose aus D-Sorbitol vorzugsweise aus einer Aminosäuresequenz, die durch eine Nukleinsäure kodiert wird, die eine Identität zu SEQ ID Nr. 6 von mindestens 80%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist. Besonders bevorzugt umfasst die Nukleinsäure, welche die erfindungsgemäße Oxidoreduktase zur Bildung von L-Sorbose aus D-Sorbitol kodiert, die Nukleinsäuresequenz SEQ ID Nr. 6 oder besteht aus dieser.

### Materialien

L-Sorbose wurde von TCI oder Sigma-Aldrich, NADPH-Tetranatriumsalz, und Methanol wurden von PanReac AppliChem (ITW Reagents), D-Glucose, Zinkchlorid, IPTG (Isopropyl-β-D-thiogalactopyranosid) und 2-Propanol wurden von Sigma-Aldrich, D-Sorbitol, Magnesiumchlorid-Hexahydrat, Kaliumdihydrogenphosphat, di-Kaliumhydrogenphosphat, NAD⁺, NADH-Dinatriumsalz, NADP⁺-Dinatriumsalz sowie Natriumdodecylsulfat (SDS) wurden von Carl Roth und Triethanolamin (TEA) wurde von Chem-Lab NV bezogen.

### Produktion der Enzyme & Herstellung der Lysate

### Allgemeines zur Expression von rekombinanten Enzymen in E. coli

Für die rekombinante Enzymproduktion in einem *Escherichia* coli-Stamm wurde zunächst das zu exprimierende Gen in einer PCR unter der Verwendung der genomischen DNA oder deren synthetisch an die Codon-Verwendung von *E. coli* angepasstes Äquivalent als Matrize zusammen mit spezifischen Oligonukleotiden, die zusätzlich Erkennungssequenzen für Restriktionsendonukleasen tragen, amplifiziert und aus dem Reaktionsgemisch isoliert. Nach dem Nukleinsäure-Verdau mit den Restriktionsenzymen Sphl und Hindlll wurde das für das Target-Enzym kodierende Genfragment in das mit Sphl und Hindlll geschnittene Rückgrat des Expressionsvektors pQE70-Kan ligiert. Das Ligationsprodukt wurde in chemisch kompetente E. coli-Zellen Top10F transformiert und die entstandenen Kolonien wurden für die Plasmid-Isolierung und Restriktionsanalyse benutzt.

Das Ergebnis des Klonierungs-Schritts wurde mittels Restriktionsenzym-Verdau und DNA-Sequenzierung überprüft. Das resultierende Konstrukt trägt das Target-Gen unter dem IPTGinduzierbaren T5-Promotor.

Für die Überexpression des Enzymes in *E. coli* wurde das resultierende Expressionsplasmid in die kompetenten Expressionszellen RB791 transformiert. Nach 24 h Inkubation bei 37 °C wurden entstandene Kolonien für die Expressionstests in LB-Medium angeimpft.

Am nächsten Tag wurden damit Expressionskulturen mit einer optische Dichte OD₅₅₀ von 0,02 angeimpft und bei 37 °C geschüttelt, bis eine OD₅₅₀ von 0,3 erreicht wurde. Anschließend wurde die Temperatur auf 25 °C gesenkt und die Kulturen wurden beim Erreichen einer OD₅₅₀ von 0,5 mit 0,1 mM IPTG induziert. Nach 22 h wurden die Kulturen geerntet (vom Medium mittels Zentrifugation in Form eines Zellpellets abgetrennt) und auf die Expression des rekombinanten Enzymes mit Hilfe von SDS-Gelelektrophorese und einer Aktivitätsbestimmung (Verwendung in Use-Test oder optisch-enzymatischer Assay) analysiert.

### Herstellung von Zell-Lysaten mittels GEA-Aufschlusses

Zur Herstellung einer Zell-Suspension (Volumen > 50 ml) wurde das nach obigen Verfahren hergestellte Zellpellet in einem geeigneten Gefäß eingewogen und in Puffer (Triethanolamin (TEA) - HCl oder Kaliumphosphat-Puffer (KPP); siehe Tabelle 1 für verwendete Puffer-Systeme) gelöst. Der Massenanteil an Biomasse beträgt üblicherweise 20%, der Rest entfällt auf den Puffer.

Zum Zell-Aufschluss wurde ein Lab Homogenizer PandaPLUS 2000 von GEA verwendet (Druck ca. 1000 bar).

Die Homogenate wurden 10 min lang bei 4 °C und 16000 rpm zentrifugiert (Hermle Z446 K Zentrifuge), um die unlöslichen Zellfragmente abzutrennen und das Lysat zu erhalten.

### Herstellung von Zell-Lysaten mittels Retsch-Aufschlusses

Zur Herstellung einer Zell-Suspension wurde das nach obigen Verfahren hergestellte Zellpellet (ca. 50 mg) in einem 2 ml Eppendorf-Vial eingewogen und mit Puffer (ca. 450 µl) versetzt (Tabelle 1 für verwendete Puffer-Systeme) und mittels Vortexens gelöst. Dadurch wird eine 10%ige Zell-Suspension erhalten.

Die Suspension wurde mit Glasperlen versetzt und in einer Retsch-Mühle MM 400 (24 Hz, 10 min) aufgeschlossen. Im Anschluss wurde das Homogenat für 1 min bei 4 °C und 16000 rpm zentrifugiert (Himac Zentrifuge CT 15RE), um das Lysat zu erhalten.

**Tabelle 1. Enzymklassen, Spenderorganismen und Aufschlussbedingungen für die in den Beispielen verwendeten Enzyme.**

| **Enzymtyp (EC-Klasse)** | **katalysierte Reaktion** | **Spenderorganismus** | **Aufschlussbedingunge n** | **Literatur/SEQ ID Nr.** |
|---|---|---|---|---|
| Xylose-Reduktase I | D-Glucose → D-Sorbitol | *Thermochaetoides thermophila* DSM 1495 | GEA (20% Biomasse in 100 mM KPP pH 7) | SEQ ID Nr. 3 (NCBI Protein Datenbank: |
| (XR I) | | | | XP_006696018.1) ; SEQ ID Nr. 4 (NCBI Nukleotid Datenbank: XM_006695955.1 ) |
| Xylose-Reduktase II (XR II) | D-Glucose → D-Sorbitol | *Rasamsonia emersonii* | GEA (20% Biomasse in 100 mM KPP pH 7) | SEQ ID Nr. 1 (NCBI Protein Datenbank: ACR78268.1); Nukleotid-Sequenz: SEQ ID Nr. 2 |
| Mannitol-Dehydrogenase (MDH) | D-Sorbitol → L-Sorbose | *Sinorhizobium meliloti* | GEA (20% Biomasse in 100 mM TEA-HCl pH 7 + 2 mM MgCl₂); Retsch (10% in 100 mM TEA-HCl pH 7 + 2 mM MgCl₂) | SEQ ID Nr. 5 (NCBI Protein Datenbank: WP_010969938.1 ); SEQ ID Nr. 6 (NCBI Nukleotid Datenbank: CP002740.1; Nt 2338874 - 2340358) |
| Alkoholdehydro genase (ADH) (EC 1.1.1.2) | 2-Propanol → Aceton | *Thermoanaerobacter pseudethanolicus* | GEA (20% Biomasse in 100 mM TEA-HCl pH 7 + 0,5 mM ZnCl₂) | (NCBI Protein Database: ABY93890.1) |
| NADH-Oxidase (EC 1.6.3.4) | NADH → NAD⁺ | *Streptococcus mutans* | GEA (20% Biomasse in 100 mM KPP pH 7) | (Matsumoto et al., 1996) |

### Analytische Methoden

### High Performance Liquid Chromatography

Zur Quantifizierung von D-Glucose, L-Sorbose sowie D-Sorbitol mittels HPLC (High Performance Liquid Chromatography) wurde ein Agilent HPLC 1260 Infinity II Series System verwendet. Die Detektion erfolgte mittels eines Brechungsindex-Detektors (RI-Detektion). Zur Messung wurde eine Phenomenex Rezex RCM-Monosaccharide Ca+2 (8%) Säule mit entsprechender Vorsäule verwendet und mit 3,5% (v/v) 2-Propanol in Reinstwasser isokratisch eluiert.

### Bestimmung von Enzym-Aktivitäten (optisch-enzymatischer Assay)

Enzym-Aktivitäten in den Lysaten wurden mit einem Shimadzu UV-1900 Spektrophotometer bestimmt. Dazu wurde die Bildung bzw. der Verbrauch von NAD(P)H bei einer Wellenlänge von 340 nm über die Änderung der Absorption verfolgt. Die Messungen wurden mit 0,2 mM Kofaktor (NAD(P)⁺ oder NAD(P)H) durchgeführt. Dazu wurden 20 µl einer 10 mM Stock-Lösung des Kofaktors in einer Küvette (Greiner bio-one Semi-Micro-Küvette aus Polystyrol) vorgelegt und der gewünschte pH-Wert wurde mit 100 mM TEA-HCl-Puffer eingestellt (870 µl). 10 µl Lysat (verdünnt oder unverdünnt) und 100 µl Substrat-Lösung wurden zur Küvette zugesetzt und die Messung unmittelbar danach gestartet. Die Messungen wurden standardmäßig bei 25 °C durchgeführt. Über den Extinktionskoeffizienten von NADH bzw. NADPH bei 340 nm (*ε* = 6220 L mol⁻¹ cm⁻¹) kann die Enzym-Aktivität des Lysats in U/ml (bezogen auf das Volumen des Lysats) oder U/g (bezogen auf die zur Herstellung eingesetzte Biomasse) bestimmt werden. 1 U steht dabei für 1 µmol Substratumsatz pro Minute (1 U = 1 µmol/min = 1,67.10⁻⁸ kat).

Mit dem nachfolgenden Beispiel wird eine bevorzugte Variante des erfindungsgemäßen Verfahrens noch näher beschrieben. Die in diesem Beispiel eingesetzten Lysate wurden nach dem oben beschriebenen Verfahren hergestellt.

### Beispiel

### Herstellung von L-Sorbose aus D-Glucose

Die Reaktion wurde in einem Labfors 5 Tisch-Bioreaktor (Infors AG) durchgeführt. Als Gefäß wurde ein Glasreaktor (Volumen 3,4 I) mit aufgesetztem Rührwerk, pH-Elektrode und O₂-Sensor verwendet. Die pH-Kontrolle erfolgte durch die Zudosierung von 1M NaOH oder 1M H₂SO₄.

Zu Beginn wurden 100 ml einer D-Glucose-Lösung (500 g/l), 198,2 ml deionisiertes Wasser, 118 ml eines 200 mM TEA-HCl-Puffers (pH 8) im Reaktor vorgelegt und unter Rühren auf 50 °C gebracht.

Zum Start der Reaktion und zur Bildung von D-Sorbitol wurden 25 ml Xylose-Reduktase I-Lysat, 8 kU Alkoholdehydrogenase-Lysat, 5 ml einer 5 mM NADP⁺-Lösung sowie 50 ml 2-Propanol eingebracht.

Nach 26 h wurde der Reaktor auf 24 °C abgekühlt und zur Bildung von L-Sorbose 25 ml Mannitol-Dehydrogenase-Lysat, 10 kU NADH-Oxidase-Lysat sowie 5 ml einer 10 mM NAD⁺-Lösung zugesetzt.

Nach 72 h Gesamtlaufzeit (Umsatz: 98,0%) wurde der Reaktorinhalt auf 70 °C erhitzt, der pH-Wert auf 4 eingestellt und für 60 min bei 70 °C gerührt. Der Niederschlag wurde über eine Glasfritte (P3) abfiltriert.

Im Filtrat wurden 92,4 g/l L-Sorbose detektiert.

Während des Betriebs wurden laufend Proben von der Reaktorlösung genommen, die folgendermaßen analysiert wurden: 100 µl des Ansatzes wurden mit 200 µl Acetonitril versetzt und im Eppendorf Thermomixer bei 85 °C und 1200 rpm für 15 min inkubiert. Die Probe wurde in einer Zentrifuge kurz zentrifugiert, mit 700 µl deionisiertem Wasser versetzt, gevortext und anschließend für 5 min bei max. g zentrifugiert. 100 µl des Überstands wurden in ein HPLC-Vial mit Insert überführt und mittels HPLC (RI-Detektion) vermessen.

### Literatur

Shintani, T. (2019). Food Industrial Production of Monosaccharides Using Microbial, Enzymatic, and Chemical Methods. Fermentation, 5(2), 47. https://doi.org/10.3390/fermentation5020047
Itoh, H., & Izumori, K. (1996). Enzymatic production of L-tagatose and L-fructose from L-sorbose and L-psicose, respectively. Journal of Fermentation and Bioengineering, 81(4), 351-353. https://doi.org/10.1016/0922-338X(96)80590-3
Leang, K., Maekawa, K., Menavuvu, B. T., Morimoto, K., Granström, T. B., Takada, G., & Izumori, K. (2004). A novel enzymatic approach to the massproduction of L-galactose from L-sorbose. Journal of Bioscience and Bioengineering, 97(6), 383-388. https://doi.org/10.1016/51389-1723(04)70223-6
Vongsuvanlert, V., & Tani, Y. (1988). L-Iditol production from L-sorbose by a methanol yeast, Candida boidinii (Kloeckera sp.) No. 2201. Journal of Fermentation Technology, 66(5), 517-523. https://doi.org/10.1016/0385-6380(88)90084-2
Xu, H.-L., Zhou, X., Chen, S., Xu, S., Li, Z., Nakanishi, H., & Gao, X.-D. (2023). Rare sugar L-sorbose exerts antitumor activity by impairing glucose metabolism. Communications Biology, 6, 259. https://doi.org/10.1038/s42003-023-04638-z
Reichstein, T., & Grüssner, A. (1934). Eine ergiebige Synthese der I-Ascorbinsäure (C-Vitamin). Helvetica Chimica Acta, 17(1), 311-328. https://doi.org/10.1002/hlca.19340170136
Sugisawa, T., Hoshino, T., Masuda, S., Nomura, S., Setoguchi, Y., Tazoe, M., Shinjoh, M., Someha, S., & Fujiwara, A. (1990). Microbial Production of 2-Keto-L-Gulonic Acid from L-Sorbose and D-Sorbitol by Gluconobacter melanogenus. Agricultural and Biological Chemistry, 54(5), 1201-1209. https://doi.org/10.1080/00021369.1990.10870125
Bronnimann, C., Bodnar, Z., Hug, P., Mallat, T., & Baiker, A. (1994). Direct Oxidation of L-Sorbose to 2-Keto-L-gulonic Acid with Molecular Oxygen on Platinum- and Palladium-Based Catalysts. Journal of Catalysis, 150(1), 199-211. https://doi.org/10.1006/jcat.1994.1336
Gounder, R., & Davis, M. E. (2013). Titanium-Beta Zeolites Catalyze the Stereospecific Isomerization of D-Glucose to L-Sorbose via Intramolecular C5-C1 Hydride Shift. ACS Catalysis, 3(7), 1469-1476. https://doi.org/10.1021/cs400273c
Kwon, Y., de Jong, E., van der Waal, J. K., & Koper, M. T. M. (2015). Selective Electrocatalytic Oxidation of Sorbitol to Fructose and Sorbose. ChemSusChem, 8(6), 970-973. https://doi.org/10.1002/cssc.201402880
Zebiri, I., Balieu, S., Guilleret, A., Reynaud, R., & Haudrechy, A. (2011). The Chemistry of L-Sorbose. European Journal of Organic Chemistry, 2011(16), 2905-2910. https://doi.org/10.1002/ejoc.201001578
Liu, L., Chen, Y., Yu, S., Chen, J., & Zhou, J. (2022). Enhanced production of L-sorbose by systematic engineering of dehydrogenases in Gluconobacter oxydans. Synthetic and Systems Biotechnology, 7(2), 730-737. https://doi.org/10.1016/j.synbio.2022.02.008
Toyama, H., Soemphol, W., Moonmangmee, D., Adachi, O., & Matsushita, K. (2005). Molecular Properties of Membrane-Bound FAD-Containing D-Sorbitol Dehydrogenase from Thermotolerant Gluconobacter frateurii Isolated from Thailand. Bioscience, Biotechnology, and Biochemistry, 69(6), 1120-1129. https://doi.org/10.1271/bbb.69.1120
Soemphol, W., Toyama, H., Moonmangmee, D., Adachi, O., & Matsushita, K. (2007). L-sorbose reductase and its transcriptional regulator involved in L-sorbose utilization of Gluconobacter frateurii. Journal of Bacteriology, 189(13), 4800-4808. https://doi.org/10.1128/JB.01895-06
Yang, X.-P., Wei, L.-J., Ye, J.-B., Yin, B., & Wei, D.-Z. (2008). A pyrroloquinoline quinine-dependent membrane-bound d-sorbitol dehydrogenase from Gluconobacter oxydans exhibits an ordered Bi Bi reaction mechanism. Archives of Biochemistry and Biophysics, 477(2), 206-210. https://doi.org/10.1016/j.abb.2008.03.030
Kim, T. S., Gao, H., Li, J., Kalia, V. C., Muthusamy, K., Sohng, J. K., In-Won, K., & Lee, J.-K. (2019). Overcoming NADPH product inhibition improves D-sorbitol conversion to L-sorbose. Scientific Reports, 9, 815. https://doi.org/10.1038/s41598-018-37401-0
Sugisawa, T., Hoshino, T., & Fujiwara, A. (1991). Purification and Properties of NADPH-Linked L-Sorbose Reductase from Gluconobacter melanogenus N44-1. Agricultural and Biological Chemistry, 55(8), 2043-2049. https://doi.org/10.1271/bbb1961.55.2043
Shinjoh, M., Tazoe, M., & Hoshino, T. (2002). NADPH-Dependent L-Sorbose Reductase Is Responsible for L-Sorbose Assimilation in Gluconobacter suboxydans IFO 3291. Journal of Bacteriology, 184(3), 861-863. https://doi.org/10.1128/JB.184.3.861-863.2002
Kubota, K., Nagata, K., Okai, M., Miyazono, K., Soemphol, W., Ohtsuka, J., Yamamura, A., Saichana, N., Toyama, H., Matsushita, K., & Tanokura, M. (2011). The Crystal Structure of L-Sorbose Reductase from Gluconobacter frateurii Complexed with NADPH and L-Sorbose. Journal of Molecular Biology, 407(4), 543-555. https://doi.org/10.1016/j.jmb.2011.01.008
Kavanagh, K. L., Klimacek, M., Nidetzky, B., & Wilson, D. K. (2002). Crystal structure of Pseudomonas fluorescens mannitol 2-dehydrogenase binary and ternary complexes. Specificity and catalytic mechanism. Journal of Biological Chemistry, 277(45), 43433-43442. https://doi.org/10.1074/jbc.M206914200
Krahulec, S., Armao, G. C., Klimacek, M., & Nidetzky, B. (2011). Enzymes of mannitol metabolism in the human pathogenic fungus Aspergillus fumigatus - kinetic properties of mannitol-1-phosphate 5-dehydrogenase and mannitol 2-dehydrogenase, and their physiological implications. FEBS Journal, 278(8), 1264-1276. https://doi.org/10.1111/j.1742-4658.2011.08047.x
Kim, T.-S., Patel, S. K. S., Selvaraj, C., Jung, W.-S., Pan, C.-H., Kang, Y. C., & Lee, J.-K. (2016). A highly efficient sorbitol dehydrogenase from Gluconobacter oxydans G624 and improvement of its stability through immobilization. Scientific Reports, 6, 33438. https://doi.org/10.1038/srep33438
Lohmar, R. L. (1957). V -THE POLYOLS. In W. Pigman (Ed.), The Carbohydrates (pp. 241-298). Academic Press. https://doi.org/10.1016/B978-0-12-395709-2.50009-9
Jonas, R., & Silveira, M. M. (2004). Sorbitol can be produced not only chemically but also biotechnologically. Applied Biochemistry and Biotechnology, 118, 321-336. https://doi.org/10.1385/ABAB:118:1-3:321
Garcia, B., Orozco-Saumell, A., López Granados, M., Moreno, J., & Iglesias, J. (2021). Catalytic Transfer Hydrogenation of Glucose to Sorbitol with Raney Ni Catalysts Using Biomass-Derived Diols as Hydrogen Donors. *ACS Sustainable Chemistry* & *Engineering,* 9(44), 14857-14867. https://doi.org/10.1021/acssuschemeng.1c04957
Genchi, G., Carocci, A., Lauria, G., Sinicropi, M. S., & Catalano, A. (2020). Nickel: Human Health and Environmental Toxicology. International Journal of Environmental Research and Public Health, 17(3), 679. https://doi.org/10.3390/ijerph17030679
Barrow, K. D., Collins, J. G., Leight, D. A., Rogers, P. L., & Warr, R. G. (1984). Sorbitol production by Zymomonas mobilis. Applied Microbiology and Biotechnology, 20, 225-232. https://doi.org/10.1007/BF00250630
Zachariou, M., & Scopes, R. K. (1986). Glucose-fructose oxidoreductase, a new enzyme isolated from Zymomonas mobilis that is responsible for sorbitol production. Journal of Bacteriology, 167(3), 863-869. https://doi.org/10.1128/jb.167.3.863-869.1986
Ikemi, M., Koizumi, N., & Ishimatsu, Y. (1990). Sorbitol production in charged membrane bioreactor with coenzyme regeneration system: I. Selective retainment of NADP(H) in a continuous reaction. Biotechnology and Bioengineering, 36(2), 149-154. https://doi.org/10.1002/bit.260360207
Ikemi, M., Ishimatsu, Y., & Kise, S. (1990). Sorbitol production in charged membrane bioreactor with coenzyme regeneration system: II. Theoretical analysis of a continuous reaction with retained and regenerated NADPH. Biotechnology and Bioengineering, 36(2), 155-165. https://doi.org/10.1002/bit.260360208
Yang, J., Li, J., Men, Y., Zhu, Y., Zhang, Y., Sun, Y., & Ma, Y. (2015). Biosynthesis of L-Sorbose and L-Psicose Based on C-C Bond Formation Catalyzed by Aldolases in an Engineered Corynebacterium glutamicum Strain. Applied and Environmental Microbiology, 81(13), 4284-4294. https://doi.org/10.1128/AEM.00208-15
Yang, J., Zhu, Y., Men, Y., Sun, S., Zeng, Y., Zhang, Y., Sun, Y. &, Ma, Y. (2016). Pathway Construction in Corynebacterium glutamicum and Strain Engineering To Produce Rare Sugars from Glycerol. Journal of Agricultural and Food Chemistry, 64(50), 9497-9505. https://doi.org/10.1021/acs.jafc.6b03423
Kim, W.-K., Chun, U.-H., Park, Y.-M., Kim, C.-H., Choi, E.-S., & Rhee, S.-K. (1994). L-Sorbose production from glucose and fructose using Zymomonas mobilis and Gluconobacter suboxydans in a two-stage fed-batch reactor. Process Biochemistry, 29(4), 277-283. https://doi.org/10.1016/0032-9592(94)80069-3
Lin, B., & Tao, Y. (2017). Whole-cell biocatalysts by design. Microbial Cell Factories, 16, 106. https://doi.org/10.1186/s12934-017-0724-7
Altschul, S. F., Gish, W., Miller, W., Myers, E. W., & Lipman, D. J. (1990). Basic local alignment search tool. Journal of Molecular Biology, 215(3), 403-410. https://doi.org/10.1016/S0022-2836(05)80360-2
Henikoff, S., & Henikoff, J. G. (1992). Amino acid substitution matrices from protein blocks. Proceedings of the National Academy of Sciences of the United States of America, 89(22), 10915-10919. https://doi.org/10.1073/pnas.89.22.10915
Sambrook, J., Fritsch, E. R., & Maniatis, T. (1989). Molecular Cloning: A Laboratory Manual (2nd ed.). Cold Spring Harbor, NY: Cold Spring Harbor Laboratory Press.
Protein [Internet]. Bethesda (MD): National Library of Medicine (US), National Center for Biotechnology Information. Accession No. XP_006696018.1, NAD(P)H-dependent D-xylose reductase-like protein [Thermochaetoides thermophila DSM 1495]; [aufgerufen am 05.07.2023]. https://www.ncbi.nlm.nih.gov/protein/XP_006696018.1
Nucleotide [Internet]. Bethesda (MD): National Library of Medicine (US), National Center for Biotechnology Information. Accession No. XM_006695955.1, Chaetomium thermophilum var. thermophilum DSM 1495 NAD(P)H-dependent D-xylose reductase-like protein (CTHT_0056950), partial mRNA; [aufgerufen am 05.07.2023]. https://www.ncbi.nlm.nih.gov/nuccore/XM_006695955.1
Protein [Internet]. Bethesda (MD): National Library of Medicine (US), National Center for Biotechnology Information. Accession No. ACR78268.1, xylose reductase [Rasamsonia emersonii]; [aufgerufen am 05.07.2023]. https://www.ncbi.nlm.nih.gov/protein/ACR78268.1
Protein [Internet]. Bethesda (MD): National Library of Medicine (US), National Center for Biotechnology Information. Accession No. WP_010969938.1, mannitol dehydrogenase family protein [Sinorhizobium meliloti]; [aufgerufen am 05.07.2023]. https://www.ncbi.nlm.nih.gov/protein/WP_010969938.1
Nucleotide [Internet]. Bethesda (MD): National Library of Medicine (US), National Center for Biotechnology Information. Accession No. CP002740.1, Sinorhizobium meliloti BL225C, complete genome; [aufgerufen am 05.07.2023]. https://www.ncbi.nlm.nih.gov/nuccore/CP002740.1
Protein [Internet]. Bethesda (MD): National Library of Medicine (US), National Center for Biotechnology Information. Accession No. ABY93890.1, Alcohol dehydrogenase, zinc-binding domain protein [Thermoanaerobacter pseudethanolicus ATCC 33223]; [aufgerufen am 05.07.2023]. https://www.ncbi.n!m.nih.gov/protein/ABY93890.1
Matsumoto, J., Higuchi, M., Shimada, M., Yamamoto, Y., & Kamio, Y. (1996). Molecular Cloning and Sequence Analysis of the Gene Encoding the H2O-forming NADH Oxidase from Streptococcus mutans. Bioscience, Biotechnology, and Biochemistry, 60(1), 39-43. https://doi.org/10.1271/bbb.60.39

## Patentansprüche

1. Verfahren zur Herstellung einer wässerigen Lösung enthaltend L-Sorbose, indem D-Glucose, welche in einer wässerigen Lösung gelöst vorliegt, durch Behandeln mit einer NAD(P)H-abhängigen Oxidoreduktase *in vitro* zu D-Sorbitol reduziert wird, wonach das D-Sorbitol mit einer NAD(P)⁺-abhängigen Oxidoreduktase *in vitro* zu L-Sorbose oxidiert wird, wonach die beiden Oxidoreduktasen abgetrennt werden.

2. Verfahren nach Anspruch 1, **dadurch gekennzeichnet, dass** der durch die Reduktion entstandene oxidierte Kofaktor NAD(P)⁺ mittels einer Alkoholdehydrogenase und einem sekundären Alkohol unter Bildung eines Ketons reduziert wird.

3. Verfahren nach Anspruch 2, **dadurch gekennzeichnet, dass** der sekundäre Alkohol 2-Propanol ist.

4. Verfahren nach einem der Ansprüche 1 bis 3, **dadurch gekennzeichnet, dass** der durch die Oxidation entstandene reduzierte Kofaktor NAD(P)H mittels einer Oxidase und Sauerstoff zu NAD(P)⁺ oxidiert wird.

5. Verfahren nach einem der Ansprüche 1 bis 4, **dadurch gekennzeichnet, dass** es als Eintopf-Reaktion ohne Isolierung von Zwischenprodukten durchgeführt wird.

6. Verfahren nach einem der Ansprüche 1 bis 5, **dadurch gekennzeichnet, dass** die Oxidoreduktasen als Lysat der entsprechenden, sie bildenden Zellen vorliegen.

7. Verfahren nach einem der Ansprüche 1 bis 6, **dadurch gekennzeichnet, dass** die NAD(P)H-abhängige Oxidoreduktase zur Reduktion von D-Glucose zu D-Sorbitol eine Xylose-Reduktase ist und vorzugsweise eine Aminosäuresequenz umfasst, welche ausgewählt ist aus der Gruppe bestehend aus:
i) einer Aminosäuresequenz, die eine Identität zu SEQ ID Nr. 1 oder 3 von mindestens 80% aufweist,
ii) einer Aminosäuresequenz, die durch eine Nukleinsäure kodiert wird, die eine Identität zu SEQ ID Nr. 2 oder 4 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 2 oder 4 bindet.

8. Verfahren nach einem der Ansprüche 1 bis 7, **dadurch gekennzeichnet, dass** die NAD(P)⁺-abhängige Oxidoreduktase zur Oxidation von D-Sorbitol zu L-Sorbose eine Mannitol-Dehydrogenase ist und vorzugsweise eine Aminosäuresequenz umfasst, welche ausgewählt ist aus der Gruppe bestehend aus:
i) einer Aminosäuresequenz, die eine Identität zu SEQ ID Nr. 5 von mindestens 80% aufweist,
ii) einer Aminosäuresequenz, die durch eine Nukleinsäure kodiert wird, die eine Identität zu SEQ ID Nr. 6 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 6 bindet.

9. Verwendung einer NAD(P)H-abhängigen Oxidoreduktase zur Reduktion von D-Glucose zu D-Sorbitol eine Xylose-Reduktase, wobei die Oxidoreduktase eine Aminosäuresequenz umfasst, welche ausgewählt ist aus der Gruppe bestehend aus:
i) einer Aminosäuresequenz, die eine Identität zu SEQ ID Nr. 1 oder 3 von mindestens 80% aufweist,
ii) einer Aminosäuresequenz, die durch eine Nukleinsäure kodiert wird, die eine Identität zu SEQ ID Nr. 2 oder 4 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 2 oder 4 bindet.

10. Verwendung einer NAD(P)⁺-abhängigen Oxidoreduktase zur Oxidation von D-Sorbitol zu L-Sorbose eine Mannitol-Dehydrogenase ist und vorzugsweise eine Aminosäuresequenz umfasst, welche ausgewählt ist aus der Gruppe bestehend aus:
i) einer Aminosäuresequenz, die eine Identität zu SEQ ID Nr. 5 von mindestens 80% aufweist,
ii) einer Aminosäuresequenz, die durch eine Nukleinsäure kodiert wird, die eine Identität zu SEQ ID Nr. 6 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 6 bindet.
